# FASCICULE DE BREVET EUROPEEN

(11) **EP 0 878 550 B1**
(45) Date de publication et mention de la délivrance du brevet: **12.03.2003**
(21) Numéro de dépôt: 97410052.1
(22) Date de dépôt: 16.05.1997
(51) Int. Cl.: C12Q 1/18, C12Q 1/04

(54) **Système de validation et d'interprétation de résultats de test de sensibilité de micro-organismes à des agents antimicrobiens**
System zur Prüfung und Auswertung von Testergebnissen in der Antibiotikaempfindlichkeitstestung von Mikroorganismen
System for validation and interpretation of test results in antibiotic sensitivity testing of microorganisms

(43) Date de publication de la demande: 18.11.1998
(73) Titulaire: BIOMERIEUX, 69280 Marcy-L'Etoile (FR)
(72) Inventeur: Boeufgras, Jean-Marc, 01150 Vaux en Bugey (FR); Lazzarini, Annie, 01500 Ambronay (FR); Peyret, Michel c/o Monsieur Orange, 69004 Lyon (FR)
(74) Mandataire: de Beaumont, Michel

(56) Documents cités:
- EP-A- 0 010 846
- EP-A- 0 565 994
- WO-A-90/04646
- CORNAGLIA ET AL.: "Rapid access to pharmacokinetics data and correlation between antimicrobial susceptibility results and drug tissue distribution using a personal computer" MICROBIOLOGICA, vol. 16, 1993, BOLOGNA, pages 149-164, XP002045581

## Description

La présente invention concerne un procédé d'analyse de résultats de test de sensibilité d'une bactérie à des antibiotiques afin d'assister le médecin à prescrire un traitement. L'analyse peut s'étendre plus généralement à des tests d'agents antimicrobiens sur des micro-organismes.

Un procédé d'analyse classique consiste à réaliser sur une souche bactérienne présente dans un échantillon, par exemple le sang d'un patient, des tests d'identification et d'antibiogramme. L'identification a pour but de connaître de façon plus ou moins précise l'espèce bactérienne à laquelle appartient la souche étudiée. Elle est réalisée notamment par une observation macroscopique et microscopique, et une mise en oeuvre de tests à l'aide de réactifs biochimiques spécifiques. En général, la seule connaissance de l'espèce bactérienne ne suffit pas à prédire l'efficacité d'un antibiotique donné sur la souche étudiée. En effet, pour chaque famille d'antibiotiques, les souches d'une même espèce peuvent posséder des mécanismes de résistance différents, qui n'inactiveront pas toujours les mêmes antibiotiques au sein de la famille. L'antibiogramme consiste en la mise en présence de la souche bactérienne étudiée et de différents antibiotiques susceptibles d'être efficaces sur cette souche. Il repose sur une mesure plus ou moins précise de la Concentration Minimale Inhibitrice (CMI) de chacun des antibiotiques pour la souche étudiée, c'est-à-dire la concentration minimale en antibiotique pour laquelle la souche arrête de se développer.

Les comités d'experts établissent un premier seuil de CMI au-dessous duquel l'espèce testée est déclarée "sensible", et un deuxième seuil au-dessus duquel l'espèce est déclarée "résistante". Entre les deux seuils, l'espèce est déclarée "intermédiaire". Ce sont ces informations que l'on fournit généralement au médecin.

Avant d'être utilisé par le médecin comme base pour prescrire un traitement antibiotique, le résultat de l'antibiogramme est souvent interprété. L'objectif de cette interprétation est de déceler d'éventuelles erreurs de test, ou des risques de discordances entre le comportement de la souche étudiée devant un antibiotique donné *in vitro* lors du test et *in vivo* dans l'organisme du patient au cours du traitement. Cette démarche s'appuie le plus souvent sur des règles semi-empiriques. Par exemple, elle permet de détecter comme erroné un résultat sensible à un antibiotique lorsque la souche étudiée appartient à une espèce systématiquement résistante à cet antibiotique, ou lorsqu'elle présente une résistance à un antibiotique proche connu comme systématiquement plus actif. Dans certains cas, elle s'appuie sur la connaissance des mécanismes de résistance possibles pour l'espèce à laquelle appartient la souche étudiée.

Il est ainsi possible de corriger ou de commenter les résultats rendus pour certains antibiotiques, lorsque certains éléments font suspecter que la souche possède un mécanisme de résistance pouvant s'exprimer moins bien *in vitro* que dans l'organisme. Cette interprétation fait intervenir également des notions d'appréciation du risque pour le patient : en cas de doute pour un antibiotique, on préfère en général déclarer une souche résistante, s'il subsiste d'autres antibiotiques disponibles pour un traitement, auxquels elle a été trouvée sensible sans ambiguïté.

Les systèmes d'analyse actuels permettent d'effectuer les tests de manière automatisée et sont capables d'indiquer, pour chaque antibiotique testé, si l'espèce est résistante, intermédiaire ou sensible. Cornaglia et al. (Microbiologica (1993) vol. 16, p. 149-164) décrivent un procédé d'analyse comprenant identifier des souches bactériennes, mesurer leur sensibilité à différents agents antimicrobiens (antibiotiques) et établir des bases de données comprenant répertorier la sensiblité desdites souches bactériennes à différents antibiotiques sous la forme de valeurs discrètes de concentrations minimales inhibitrices (CMI). Par ailleurs, certains de ces systèmes permettent, notamment par la mise en oeuvre d'une base de règles, d'automatiser une partie de l'interprétation. Les bases de règles mises en oeuvre dans ces systèmes reproduisent le plus souvent les règles semi-empiriques utilisées classiquement. Or ces règles ne sont efficaces que pour détecter et corriger certains cas prédéterminés d'erreurs. Un problème réside donc dans la mise au point d'une méthode d'interprétation permettant de détecter tous types d'erreurs et d'en proposer si possible la correction.

La reconnaissance des mécanismes de résistance risquant de mal s'exprimer *in vitro* est requise pour effectuer la correction ou le commentaire des résultats. Les bases de règles mises en oeuvre dans les système actuels s'appuient sur la catégorisation en "sensible", "intermédiaire" ou "résistant", et dépendent étroitement de la liste des antibiotiques testés. Dans un grand nombre de cas, elles ne permettent pas de déterminer précisément le mécanisme de résistance.

Par ailleurs, les couples de seuils de CMI déterminant les catégories "sensible", "intermédiaire" et "résistante", étant fixés par des comités d'experts nationaux, sont susceptibles d'être modifiés dans le temps et diffèrent d'un pays à l'autre, voire parfois d'un laboratoire à l'autre dans certains pays. Il en est de même pour les recommandations concernant la conduite à tenir en cas de mise en évidence d'un mécanisme de résistance risquant de mal s'exprimer *in vitro*. Ainsi, on doit prévoir des bases de règles différentes correspondant aux choix interprétatifs des différents comités d'experts nationaux, et permettant une adaptation des règles en fonction des laboratoires. De telles bases de règles sont particulièrement complexes et leur réalisation représente un travail considérable.

Un objet de la présente invention est de prévoir un procédé d'analyse de tests de sensibilité qui soit indépendant des choix interprétatifs des comités d'experts, qui permette de détecter et de corriger des erreurs sans avoir à prévoir à l'avance les cas d'erreurs à traiter, et qui permette de fournir, dans la plupart des cas, une indication précise des mécanismes de résistance d'une souche testée pour les différentes familles d'antibiotiques.

Ces objets sont atteints grâce à un procédé d'analyse de résultats d'un test de sensibilité d'un micro-organisme à des agents antimicrobiens, le test consistant à identifier sommairement l'espèce à laquelle appartient un micro-organisme et à mesurer les concentrations minimales inhibitrices (CMI) de plusieurs agents antimicrobiens pour ce micro-organisme. Le procédé utilise une base de données répertoriant des espèces de micro-organisme ainsi que leurs mécanismes de résistance devant différents agents antimicrobiens, et contenant, pour chaque espèce et chaque mécanisme de résistance, des paramètres caractéristiques de distributions statistiques de CMI pour un groupe d'agents antimicrobiens.

Selon un mode de réalisation de la présente invention, le procédé comprend les étapes consistant à extraire de la base de données les paramètres des distributions associées aux mécanismes de résistance de l'espèce identifiée et aux agents antimicrobiens utilisés pour effectuer le test ; à confronter les CMI mesurées lors du test aux paramètres extraits ; et à indiquer que le test est valide lorsque les CMI mesurées correspondent aux paramètres extraits associés à au moins un mécanisme de résistance prédéterminé de l'espèce identifiée.

Selon un mode de réalisation de la présente invention, le procédé indique le mécanisme de résistance prédéterminé.

Selon un mode de réalisation de la présente invention, lorsque le test n'est pas valide, le procédé détermine des corrections à effectuer sur au moins une des CMI mesurées, le choix des corrections répondant à des critères d'optimalité prédéterminés.

Selon un mode de réalisation de la présente invention, le procédé comprend, lorsque le test n'est pas valide, les étapes consistant à extraire de la base de données les paramètres des distributions de CMI associés aux mécanismes de résistance d'autres espèces et aux agents antimicrobiens utilisés pour effectuer le test ; à confronter les CMI mesurées aux paramètres extraits ; et à déterminer la ou les espèces pour lesquelles au moins un mécanisme de résistance, par famille d'agents antimicrobiens testés, est identifiable sur la base des CMI mesurées.

Selon un mode de réalisation de la présente invention, la base de données contient des informations indiquant, pour des espèces à mécanisme de résistance donné et pour des agents antimicrobiens donnés, une résistance *in vivo* pouvant être supérieure à la résistance *in vitro.*

Selon un mode de réalisation de la présente invention, les paramètres de distribution stockés dans la base de données comprennent les classes de valeurs de CMI et les effectifs normalisés, le procédé comprenant, pour un agent antimicrobien non testé, les étapes consistant à extraire de la base de données les classes et les effectifs associés au mécanisme de résistance prédéterminé et à l'agent antimicrobien non testé ; à retenir les classes pour lesquelles les effectifs dépassent un seuil prédéterminé ; à confronter les classes retenues à deux seuils de CMI normalisés définissant des catégories "sensible", "intermédiaire" et "résistant" d'un micro-organisme ; et à indiquer les catégories situées de part et d'autre de chacun des seuils normalisés se trouvant dans les classes retenues.

Ces objets, caractéristiques et avantages, ainsi que d'autres de la présente invention seront exposés en détail dans la description suivante de modes de réalisation particuliers faite à titre non-limitatif en relation avec les figures jointes parmi lesquelles :
la figure 1 illustre un exemple de contenu de base de données utilisée par le procédé selon l'invention ;
la figure 2 illustre un premier exemple d'analyse selon l'invention permettant d'indiquer sans ambiguïté le mécanisme de résistance d'une espèce testée ;
la figure 3 illustre un cas de test erroné et une proposition de correction de mesure fournie par le procédé selon l'invention ;
la figure 4 illustre un cas de test erroné et une proposition de correction de l'identification d'espèce ;
la figure 5 illustre une correction de déclaration de sensibilité à partir d'informations quant à une résistance supérieure *in vivo* de l'espèce testée ; et
la figure 6 illustre une déclaration fournie par le procédé selon l'invention à propos d'un antibiotique non testé.

Le procédé selon l'invention utilise une base de données répertoriant des espèces de micro-organisme avec leurs mécanismes de résistance aux différentes familles d'agents antimicrobiens, et des agents antimicrobiens. Pour chaque agent antimicrobien et chaque mécanisme de résistance, la base de données stocke des paramètres caractéristiques d'une distribution statistique de Concentrations Minimales Inhibitrices (CMI). Ces paramètres peuvent être par exemple une moyenne et un écart-type, ou les limites inférieure et supérieure de la distribution et une information sur sa forme, ou encore les effectifs normalisés de chaque classe de valeurs. Chaque distribution statistique est le résultat de tests effectués sur un grand nombre d'échantillons d'individus de même espèce et de même mécanisme de résistance, c'est-à-dire sur une population représentative de l'espèce et du mécanisme de résistance.

Les mécanismes de résistance sont caractérisés par leur spectre d'inactivation sur les antibiotiques d'une même famille et n'inactivent pas les antibiotiques des autres familles. Une famille est constituée d'antibiotiques présentant des structures biochimiques et des modes d'action apparentés. Par conséquent, la base de données ne stocke, pour une espèce donnée et un mécanisme de résistance donné, que les distributions statistiques de CMI associées à des antibiotiques d'une seule famille.

Le procédé selon l'invention est destiné à être mis en oeuvre dans un système d'analyse informatisé, couplé ou non à un système automatisé pour la réalisation d'antibiogrammes. Un logiciel pour mettre en oeuvre le procédé selon l'invention et la base de données pourront avantageusement remplacer le logiciel et la base de règles de systèmes d'analyse existants.

Les figures représentent des histogrammes symbolisant des distributions d'effectifs normalisés des différentes classes de valeurs de CMI (distributions de CMI). L'effectif normalisé d'une classe est, par exemple, le rapport de l'effectif de la classe à l'effectif de la classe la plus nombreuse. Les classes de valeurs de CMI sont représentées sans échelle et croissent de la gauche vers la droite. Chaque barre d'histogramme illustre le nombre d'individus (souches microbiennes) inhibés par la concentration correspondante en antibiotique, ce nombre étant décompté sur la population non inhibée par la concentration immédiatement inférieure. A la limite inférieure d'une distribution de CMI, les individus les plus sensibles commencent à être affectés par l'antibiotique correspondant. A la limite supérieure de la distribution, les derniers individus les plus résistants sont affectés.

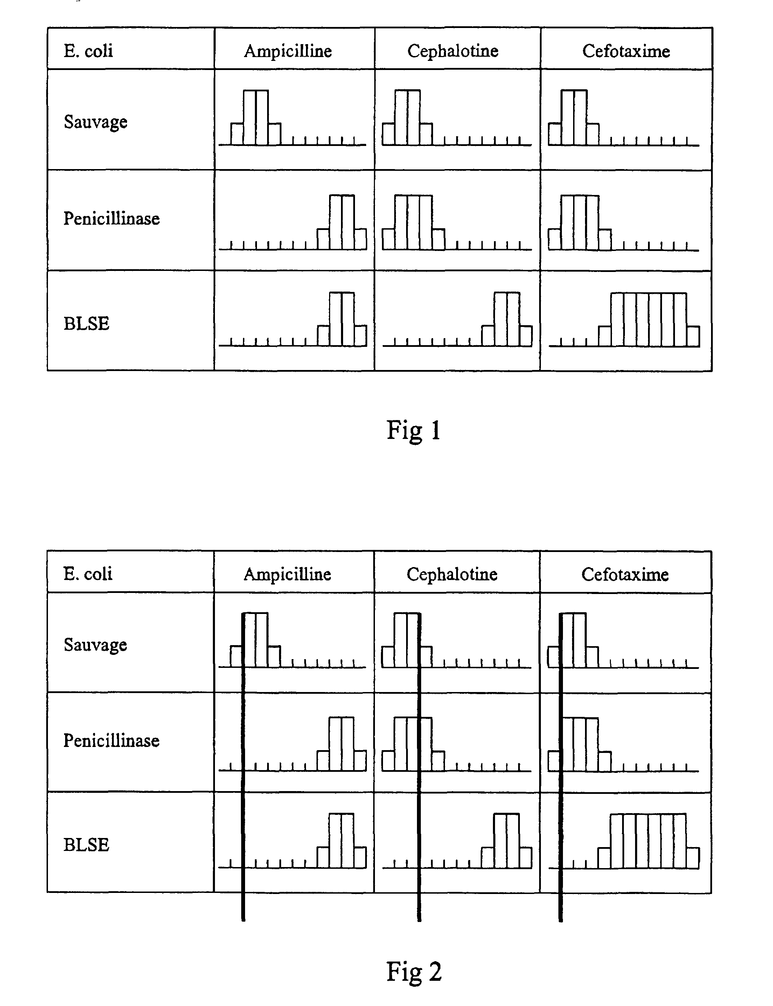

La figure 1 illustre un exemple d'extrait de la base de données. Pour l'espèce *Escherichia coli* et la famille d'antibiotiques béta-lactamines, on répertorie les mécanismes de résistance "sauvage", "pénicillinase", et "BLSE" (béta-lactamase à spectre étendu). Parmi les antibiotiques de la famille des béta-lactamines, on a illustré l'Ampicilline, la Cephalotine et le Cefotaxime.

On constate que les souches possédant le mécanisme sauvage sont plutôt sensibles aux trois antibiotiques, que celles possédant le mécanisme pénicillinase sont résistantes à l'Ampicilline et sensibles aux deux autres antibiotiques, et finalement que celles possédant le mécanisme BLSE sont plutôt résistantes aux trois antibiotiques.

La figure 2 illustre une étape principale du procédé selon l'invention. Lors d'un test bactériologique classique, on réalise une identification plus ou moins précise de l'espèce à laquelle appartient la souche étudiée, par exemple à l'aide de réactifs biochimiques d'identification. Cette identification fournit, par exemple, l'espèce *Escherichia coli*. Simultanément, on réalise un test d'antibiogramme avec un certain nombre d'antibiotiques. Ce test d'antibiogramme fournit une mesure de la CMI de la souche étudiée à différents antibiotiques, ou permet de situer cette CMI dans un intervalle donné. Dans cet exemple, on utilise l'Ampicilline, la Céfalotine et le Cefotaxime.

Une fois que les tests ont été effectués, une première étape du procédé consiste à extraire de la base de données les distributions de CMI associées aux mécanismes de résistance de l'espèce identifiée et aux antibiotiques testés. Les CMI mesurées, fournies par les antibiogrammes et représentées par des barres verticales dans les figures, sont alors comparées aux distributions de CMI extraites. Cette comparaison peut être réalisée, par exemple, en faisant correspondre à chaque valeur de CMI mesurée l'effectif normalisé de la classe de valeurs correspondante dans la distribution. Cet effectif normalisé reflète l'adéquation de la CMI mesurée à la distribution extraite de la base de données.

Les effectifs normalisés correspondants sont ensuite agrégés par mécanisme de résistance (par exemple en faisant la moyenne ou le produit des effectifs normalisés), pour l'ensemble des antibiotiques testés d'une même famille. Cette agrégation fournit un indice synthétique reflétant l'adéquation des CMI mesurées pour ces antibiotiques à chaque mécanisme de résistance.

Si cet indice synthétique a une valeur suffisamment élevée pour l'un des mécanismes de résistance, ce mécanisme de résistance est celui que l'on cherche à identifier et le test est valide.

L'exemple simplifié de la figure 2 permet de constater que le mécanisme de résistance à identifier est le mécanisme "sauvage", ceci du fait que c'est le seul mécanisme pour lequel chaque CMI mesurée correspond à une distribution associée au mécanisme sauvage.

Dans le cas où plusieurs mécanismes de résistance présentent un indice suffisamment élevé, on ne conservera, de préférence, que celui ou ceux dont les indices sont les plus élevés.

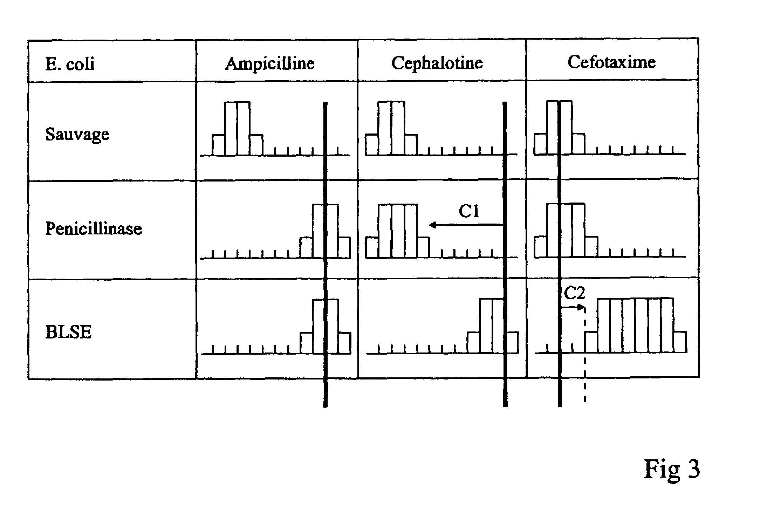

La figure 3 illustre un cas, dans le cadre de l'exemple de la figure 2, où les CMI mesurées ne permettent d'identifier aucun mécanisme de résistance. En effet, pour chaque mécanisme de résistance, au moins une des CMI mesurées est en dehors de la distribution de CMI correspondante.

Dans ce cas, on indique que le test n'est pas valide. Le procédé permet alors de proposer une correction pour une ou plusieurs des CMI mesurées, en recherchant une correction optimale selon un certain nombre de critères. On cherche notamment à minimiser le nombre d'antibiotiques corrigés, à minimiser le nombre de corrections à la baisse, à minimiser l'ampleur des corrections, à maximiser le niveau d'adéquation des CMI non corrigées aux mécanismes de résistance utilisés, à maximiser la fréquence avec laquelle ces mécanismes de résistance peuvent être rencontrés. Cette optimisation peut être réalisée en pondérant les différents critères, ou en les hiérarchisant.

Dans l'exemple de la figure 3, le mécanisme "sauvage" est exclu car il faudrait corriger deux CMI mesurées sur trois. On cherche dans cet exemple un mécanisme de résistance pour lequel il suffit de corriger une seule CMI mesurée, de sorte que toutes les CMI mesurées correspondent aux distributions de CMI associées à ce mécanisme de résistance.

Si le mécanisme de résistance était "pénicillinase", la CMI mesurée pour la Cephalotine devrait être décalée vers la gauche d'une valeur C1, c'est-à-dire diminuée, pour atteindre la limite supérieure de la distribution de CMI manquante.

Si le mécanisme de résistance était "BLSE", la CMI mesurée pour le Cefotaxime devrait être décalée vers la droite d'une valeur C2, c'est-à-dire augmentée, pour atteindre la limite inférieure de la distribution de CMI manquante.

C'est cette dernière correction C2 que l'on préférera, essentiellement pour des raisons de sécurité. En effet, la correction C2 s'effectue à la hausse, c'est-à-dire que l'on déclare l'espèce plus résistante au Cefotaxime qu'elle ne semble l'être au vu des valeurs de CMI mesurées. On préférera toujours une correction à la hausse à une correction à la baisse. Bien entendu, parmi plusieurs corrections possibles, on préférera celle de plus faible valeur.

Si l'on teste plus de trois antibiotiques, on pourra proposer des corrections pour plus d'une valeur mesurée, le nombre de valeurs corrigées devant rester limité.

Le procédé permet également de proposer une correction de l'espèce identifiée. En effet, le processus d'identification de l'espèce comporte toujours un certain risque d'erreur. Le niveau de ce risque dépend en partie de la méthode d'identification employée, et en partie des espèces en cause, certaines espèces présentant, avec la plupart des méthodes utilisables, un risque non négligeable d'être confondues avec des espèces proches.

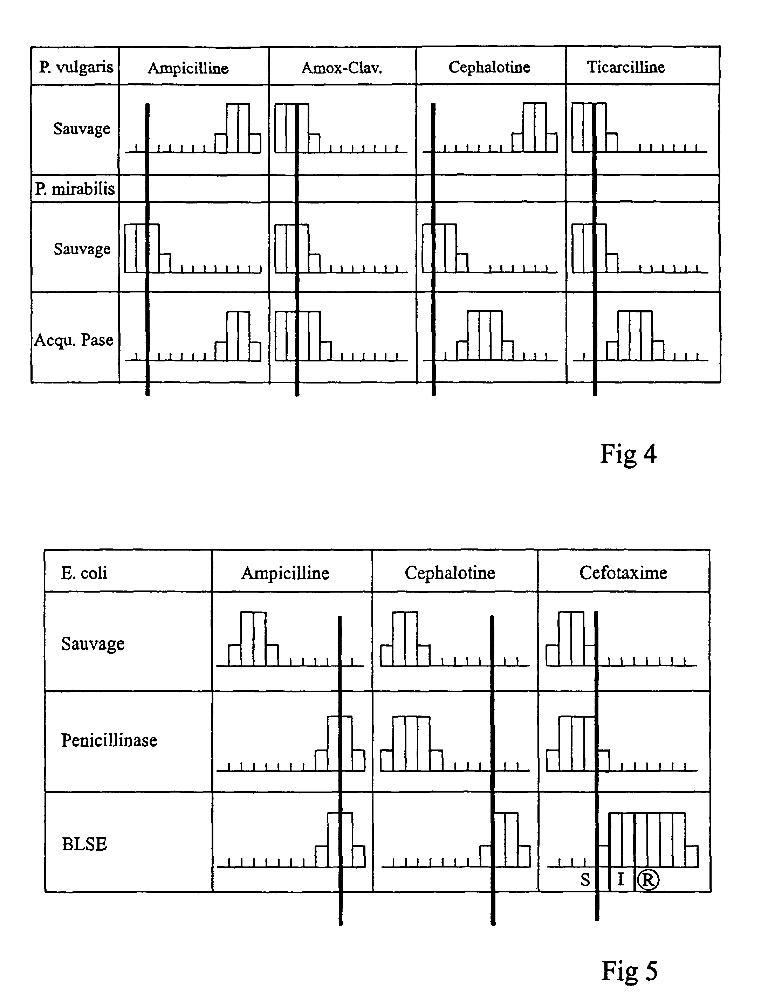

La figure 4 illustre une telle proposition de correction de l'identification de l'espèce. L'espèce a été identifiée initialement comme *"Proteus vulgaris",* et les antibiotiques testés sont l'Ampicilline, l'Augmentin (Amoxicilline-Acide clavulanique), la Cephalotine et la Ticarcilline.

Pour cette espèce, aucun mécanisme de résistance ne correspond aux CMI mesurées (la figure ne représente que le mécanisme "sauvage"). Par contre, il s'avère que la base de données répertorie une espèce, *Proteus mirabilis,* dont l'un des mécanismes de résistance, sauvage, correspond parfaitement aux CMI mesurées. Dans ce cas, le système peut proposer l'espèce *Proteus mirabilis à* mécanisme de résistance "sauvage".

Dans la plupart des cas, on teste d'autres familles d'antibiotiques (les figures n'illustrent qu'une famille). Dans ces cas, avant de proposer une telle correction d'espèce, le système cherche à identifier des mécanismes de résistance supplémentaires pour les autres familles d'antibiotiques testés. Une espèce n'est proposée que si on parvient à identifier au moins un mécanisme de résistance par famille différente d'antibiotiques. De préférence, le système indiquera le ou les mécanismes de résistance identifiés pour l'espèce proposée en correction.

Dans l'exemple de la figure 4, on remarquera que l'espèce proposée en correction est du même genre que l'espèce initialement identifiée. De manière générale, le système proposera en correction une espèce présentant un risque de confusion avec l'espèce initialement identifiée.

Pour proposer ce type de correction, la base de données peut contenir notamment des regroupements d'espèces présentant un risque de confusion. Lorsqu'aucun mécanisme de résistance n'est reconnu pour l'espèce initialement identifiée, le système recherche une espèce en priorité dans le regroupement correspondant.

La figure 5 illustre l'utilisation d'informations de discordance entre les sensibilités *in vitro* et *in vivo*, que l'on peut également stocker dans la base de données.

Dans l'exemple de la figure 5, on teste de nouveau l'espèce *Escherichia coli* avec l'Ampicilline, la Cephalotine et le Cefotaxime.

Les CMI mesurées permettent d'identifier le mécanisme de résistance "BLSE". La CMI mesurée pour le Cefotaxime indique que l'espèce est plutôt sensible. Or, des recherches ont montré que les souches possédant un mécanisme de résistance BLSE peuvent être plus résistantes au Cefotaxime *in vivo* que *in vitro.* Certains comités d'experts préconisent donc que l'espèce soit déclarée résistante au Cefotaxime, même si les mesures révèlent qu'elle est sensible *in vitro.* Ces préconisations peuvent en plus tenir compte de la catégorie de sensibilité initialement définie en comparant les CMI aux seuils établis par les comités d'experts pour définir les catégories "sensible" et "résistante". Ainsi, pour certains antibiotiques et certains mécanismes de résistance, il peut être préconisé de transformer en intermédiaire une catégorie initialement calculée comme sensible, et de conserver des catégories initialement calculées comme résistantes.

Ainsi, pour chaque antibiotique et chaque mécanisme de résistance d'une espèce, la base de données peut contenir une telle information de résistance *in vivo,* ce qui permettra au système d'en tenir compte dès que l'antibiotique testé et le mécanisme de résistance identifié correspondent. Le système contient également les règles permettant de combiner ces informations aux catégories de sensibilité définies à partir des seuils.

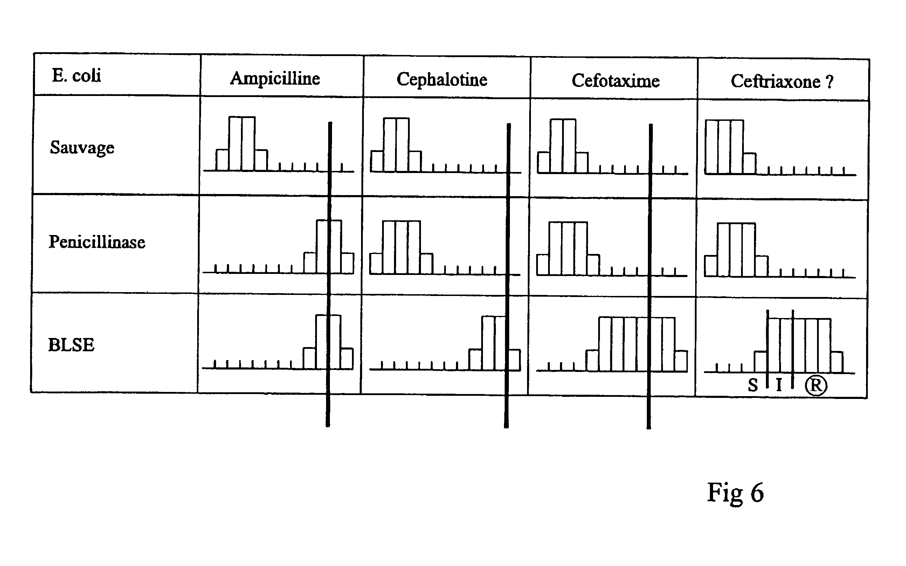

La figure 6 illustre l'utilisation des informations de la base de données pour fournir des indications supplémentaires. Comme dans l'exemple précédent, on teste une souche identifiée à l'espèce *Escherichia coli* avec l'Ampicilline, la Cephalotine et le Cefotaxime. Le procédé révèle le mécanisme de résistance BLSE.

On pourrait souhaiter des renseignements sur d'autres antibiotiques couramment utilisés pour traiter les infections par *Escherichia coli*, tels que le Ceftriaxone.

De la distribution des CMI du Ceftriaxone pour les souches de l'espèce *Escherichia coli* possédant un mécanisme de résistance BLSE, on déduit la plage dans laquelle se situe probablement la CMI de la souche étudiée pour cet antibiotique, en ne retenant que les classes de valeurs de CMI pour lesquelles l'effectif normalisé dépasse un seuil prédéterminé. En première intention, la catégorie de sensibilité de la souche étudiée peut être déterminée en confrontant les classes retenues aux deux seuils établis par les comités d'experts pour définir les catégories "sensible", "intermédiaire" et "résistante'!. Ainsi, le système indiquera les catégories situées de part et d'autre de chaque seuil compris dans les classes retenues.

Dans l'exemple de la figure 6, on retient toutes les classes de la distribution associée au mécanisme BLSE et au Ceftriaxone. Les deux seuils de CMI sont indiqués par des traits gras et sont compris tous les deux dans les classes retenues. Le système indique alors les trois catégories "sensible", "intermédiaire" et "résistante".

De plus, comme pour le Cefotaxime, des recherches ont révélé que les *Escherichia coli* à mécanisme BLSE peuvent s'avérer résistantes au Ceftriaxone *in vivo.* Ainsi, certains experts préconisent de déclarer cette bactérie résistante au Ceftriaxone, quel que soit le résultat de la détermination de la CMI *in vitro.* Le système peut donc indiquer que la bactérie est résistante au Ceftriaxone, même si le Ceftriaxone n'a pas été testé.

De façon générale, le système pourra fournir une indication sur le niveau de résistance probable de la souche aux antibiotiques non testés, en se basant sur la distribution de CMI pour cet antibiotique et le mécanisme de résistance reconnu, et sur les informations de résistance *in vivo* correspondantes.

## Revendications

1. Procédé d'identification du mécanisme de résistance d'un micro-organisme, ce mécanisme de résistance étant associé à une famille d'agents antimicrobiens, comprenant les étapes suivantes :
utiliser une base de données répertoriant diverses espèces de micro-organismes, chaque espèce étant susceptible de présenter différents mécanismes de résistance pour ladite famille d'agents antimicrobiens, ladite base de données contenant, pour chaque espèce et pour chaque mécanisme de résistance, des paramètres caractéristiques de distributions statistiques de concentrations minimales inhibitrices (CMI) pour lesdits agents antimicrobiens ;
identifier sommairement l'espèce à laquelle appartient le micro-organisme ;
mesurer les CMI associées à ce micro-organisme pour plusieurs agents antimicrobiens ; et
comparer les CMI mesurées aux informations stockées dans la base de données, d'où il résulte que le mécanisme de résistance est directement identifié.

2. Procédé selon la revendication 1, **caractérisé en ce qu'**il comprend les étapes suivantes :
- extraire de la base de données les paramètres des distributions associées aux mécanismes de résistance de l'espèce identifiée et aux agents antimicrobiens utilisés pour effectuer le test ;
- confronter les CMI mesurées lors du test aux paramètres extraits ; et
- indiquer que le test est valide lorsque les CMI mesurées correspondent aux paramètres extraits associés à au moins un mécanisme de résistance prédéterminé de l'espèce identifiée.

3. Procédé selon la revendication 2, **caractérisé en ce qu'**il comprend l'étape consistant à indiquer le mécanisme de résistance prédéterminé.

4. Procédé selon la revendication 2, **caractérisé en ce qu'**il comprend, lorsque le test n'est pas valide, une étape consistant à déterminer des corrections à effectuer sur au moins une des CMI mesurées, le choix des corrections répondant à des critères d'optimalité prédéterminés.

5. Procédé selon la revendication 2, **caractérisé en ce qu'**il comprend, lorsque le test n'est pas valide, les étapes suivantes :
- extraire de la base de données les paramètres des distributions de CMI associés aux mécanismes de résistance d'autres espèces et aux agents antimicrobiens utilisés pour effectuer le test ;
- confronter les CMI mesurées aux paramètres extraits ; et
- déterminer la ou les espèces pour lesquelles au moins un mécanisme de résistance, par famille d'agents antimicrobiens testés, est identifiable sur la base des CMI mesurées.

6. Procédé selon la revendication 2, **caractérisé en ce que** la base de données contient des informations indiquant, pour des espèces à mécanisme de résistance donné et pour des agents antimicrobiens donnés, une résistance *in vivo* pouvant être supérieure à la résistance *in vitro.*

7. Procédé selon la revendication 2, **caractérisé en ce que** les paramètres de distribution stockés dans la base de données comprennent les classes de valeurs de CMI et les effectifs normalisés, le procédé comprenant, pour un agent antimicrobien non testé, les étapes suivantes :
- extraire de la base de données les classes et les effectifs associés au mécanisme de résistance prédéterminé et à l'agent antimicrobien non testé ;
- retenir les classes pour lesquelles les effectifs dépassent un seuil prédéterminé ;
- confronter les classes retenues à deux seuils de CMI normalisés définissant des catégories "sensible", "intermédiaire" et "résistant" d'un micro-organisme ; et
- indiquer les catégories situées de part et d'autre de chacun des seuils normalisés se trouvant dans les classes retenues.

## Claims

1. A method for identifying the resistance mechanism of a micro-organism, said resistance mechanism being associated with a family of antimicrobial agents, comprising the following steps:
using a database indexing different micro-organism species, each species being liable to have different resistance mechanisms against said family of antimicrobial agents, said database containing, for each of said species and each of said resistance mechanisms, parameters characteristic of statistic minimum inhibitory concentrations (MIC) distributions for said antimicrobial agents;
roughly identifying the species to which said micro-organism belongs;
measuring the MICs associated with said micro-organism for several antimicrobial agents; and
comparing the measured MICs associated with said micro-organism to the information stored in said database, whereby said resistance mechanism is directly identified.

2. The method of claim 1, **characterized in that** it includes the steps of:
- extracting from the database the parameters of the distributions associated with the resistance mechanisms of the identified species and with the antimicrobial agents used to perform the test;
- comparing the MICs measured during the test with the extracted parameters; and
- indicating that the test is valid when the measured MICs correspond to the extracted parameters associated with at least one predetermined resistance mechanism of the identified species.

3. The method of claim 2, **characterized in that** it includes the step of indicating the predetermined resistance mechanism.

4. The method of claim 2, **characterized in that** it includes, when the test is not valid, a step of determining corrections to be performed on at least one of the measured MICs, the choice of the corrections fulfilling predetermined optimality criteria.

5. The method of claim 2, **characterized in that** it includes, when the test is not valid, the steps of:
- extracting from the database the parameters of the MIC distributions associated with the resistance mechanisms of other species and with the antimicrobial agents used for the testing;
- comparing the measured MICs with the extracted parameters; and
- determining the species for which at least one resistance mechanism, per tested antimicrobial agent family, is identifiable based on the measured MICs.

6. The method of claim 2, **characterized in that** the database contains information indicating, for species with a given resistance mechanism and for given antimicrobial agents, an *in vivo* resistance which may be higher than the *in vitro* resistance.

7. The method of claim 2, **characterized in that** the distribution parameters stored in the database include the classes of MIC values and the normalized absolute frequencies, the method including, for an untested antimicrobial agent, the steps of:
- extracting from the database the classes and absolute frequencies associated with the predetermined resistance mechanism and the untested antimicrobial agent;
- keeping the classes for which the absolute frequencies exceed a predetermined threshold;
- confronting the kept classes with two normalized MIC thresholds defining "susceptible", "intermediate", and "resistant" categories of a micro-organism; and
- indicating the categories located on either side of each of the normalized thresholds located in the retained classes.

## Patentansprüche

1. Verfahren zur Identifizierung des Resistenzmechanismus eines Mikroorganismus, wobei dieser Resistenzmechanismus einer Familie von Antimikroben-Agenten zugeordnet ist, und wobei dieses Verfahren die folgenden Stufen bzw. Schritte umfasst:
Verwendung einer Datenbasis, welche verschiedene Spezies von Mikroorganismen inventarisiert, deren jede jeweils unterschiedliche Resistenzmechanismen gegenüber der genannten Familie antimikrobieller Agentien aufweist, wobei die genannte Datenbasis für jede Spezies und für jeden Resistenzmechanismus charakteristische Parameter statistischer Verteilungen minimaler Inhibitions-Konzentrationen (MIC, minimum inhibitory concentrations) für die genannten antimikrobieller Agenten enthält;
Summarische Identifizierung der Spezies, zu welcher der Mikroorganismus gehört;
Messen der diesen Mikroorganismen zugeordneten minimalen Inhibitions-Konzentrationen bezüglich mehreren antimikrobiellen Agentien; sowie
Vergleichen der gemessenen MIC-Werte mit den in der Datenbasis gespeicherten Informationen, woraus als Ergebnis die direkte Identifizierung des Resistenzmechanismus folgt.

2. Verfahren nach Anspruch 1, **dadurch gekennzeichnet, daß** es die folgenden Schritte bzw Stufen umfaßt:
- aus der Datenbasis werden die Parameter der Verteilungen extrahiert, welche den Resistenzmechanismen der identifizierten Spezies und den zur Durchführung des Tests verwendeten antimikrobiellen Agentien zugeordnet sind;
- Gegenüberstellen der bei dem Test gemessenen MIC-Werte und der extrahierten Parameter;
- Anzeigen der Gültigkeit des Tests, wenn die gemessenen MIC-Werte den wenigstens einem vorbestimmten Resistenzmechanismus der identifizierten Spezies zugeordneten extrahierten Parametern entsprechen.

3. Verfahren nach Anspruch 2, **dadurch gekennzeichnet, daß** es den Verfahrensschritt enthält, welcher darin besteht, den vorbestimmten Resistenzmechanismus anzuzeigen.

4. Verfahren nach Anspruch 2, **dadurch gekennzeichnet, daß** es bei nicht gültigem Test eine Verfahrensstufe bzw. einen Verfahrensschritt umfaßt, welcher darin besteht, an wenigstens einem der gemessenen MIC-Werte vorzunehmende Korrekturen zu bestimmen, wobei die Wahl der Korrekturen vorgegebenen Optimalitätskriterien entspricht.

5. Verfahren nach Anspruch 2, **dadurch gekennzeichnet, daß** es bei nicht gültigem Test die folgenden Verfahrensschritte bzw. -stufen umfaßt:
- aus der Datenbasis werden die Parameter der MIC-Verteilungen extrahiert, welche den Resistenzmechanismen anderer Spezies und den für die Durchführung des Tests verwendeten antimikrobiellen Agentien zugeordnet sind;
- Gegenüberstellen der gemessenen MIC-Werte und der extrahierten Parameter;
- Bestimmen der einen oder mehreren Species, für welche wenigstens ein Resistenzmechanismus je Familie getesteter antimikrobieller Agentien auf der Grundlage der gemessenen MIC-Werte identifizierbar ist.

6. Verfahren nach Anspruch 2, **dadurch gekennzeichnet, daß** die Datenbasis Informationen enthält, welche für Spezies mit gegebenem Resistenzmechanismus und für gegebene antimikrobielle Agentien eine in vivo- Resistenz anzeigen, die größer als die in vitro-Resistenz sein kann.

7. Verfahren nach Anspruch 2, **dadurch gekennzeichnet daß** die in der Datenbasis gespeicherten Verteilungs-Parameter Klassen von MIC-Werten und die normalisierten bzw. standadisierten Bestands- bzw. Häufigkeitswerte umfassen, und daß das Verfahren, für ein nicht getestes antimikrobielles Agens, die folgenden Verfahrensstufen bzw. -schritte umfasst:
- aus der Datenbasis werden die dem vorgegebenen Resistenzmechanismus und dem nicht- getesteten antimikrobiellen Agens zugeordneten Klassen und Bestands- bzw. Häufigkeitswerte extrahiert;
- Festhalten der Klassen, für welche die Häufigkeitswerte eine vorgegebene Schwelle übersteigen;
- Gegenüberstellen der festgehaltenen Klassen mit zwei Schwellwerten normalisierter MIC-Werte, welche Kategorien eines Mikroorganismus gemäß Kriterien 'empfindlich', 'Zwischenfall' sowie 'resistent' definieren; sowie
- Anzeige der beidseits jeder der normalisierten Schwellen gelegenen Kategorien, die sich in den festgehaltenen Klassen befinden.
